# EUROPEAN PATENT SPECIFICATION

(11) **EP 4 429 595 B1**
(45) Date of publication and mention of the grant of the patent: **16.09.2026**
(21) Application number: 21839247.0
(22) Date of filing: 10.11.2021
(51) Int. Cl.: A61F 2/24

(54) **ARTICULATED PROSTHESIS FOR A TRICUSPID OR MITRAL VALVE AND RELATED CATCHING DEVICE**
GELENKPROTHESE FÜR EINE TRIKUSPIDAL- ODER MITRALKLAPPE UND ZUGEHÖRIGE FANGVORRICHTUNG
PROTHÈSE ARTICULÉE POUR VALVULE TRICUSPIDE OU MITRALE ET DISPOSITIF DE CAPTURE ASSOCIÉ

(43) Date of publication of application: 18.09.2024
(73) Proprietor: STAR TRIC S.R.L., 20121 Milano (IT)
(72) Inventor: DE BONIS, Michele, 20063 Cernusco Sul Naviglio MI (IT); ALFIERI, Ottavio, 25121 Brescia BS (IT); LAPENNA, Elisabetta, 20063 Cernusco Sul Naviglio MI (IT); ZANOTTI, Daniele, 32040 Comelico Superiore BL (IT); GARD, Marco, 10031 Borgomasino TO (IT); PASSANANTE, Eugenio Maria, 20147 Milano MI (IT)
(74) Representative: Barbaro, Gaetano
(86) International application number: PCT/IB2021/060386
(87) International publication number: WO 2023/084270

(56) References cited:
- US-A1- 2019 000 613
- US-A1- 2019 365 529
- US-B1- 10 123 873

## Description

### TECHNICAL FIELD

This disclosure relates to the repair of heart valves showing regurgitation. More particularly, the invention relates to an apparatus suitable for a less invasive repair of a heart valve using an articulated prosthesis of a catching device, which can be positioned through a catheter, for the flaps of a tricuspid or mitral valve, as defined in claim 1.

### BACKGROUND

The most common type of tricuspid valve dysfunction is functional tricuspid regurgitation (TR), which is mainly due to dilation of the tricuspid annulus after the dilation of the right ventricle. Later in the course of the disease, a "tethering" of the tricuspid flaps may also take place due to the dislocation of the papillary muscles within the remodeled right ventricle. When functional TR is due to both severe annular dilation and flap chaining, annuloplasty alone is unlikely to be effective. Similarly, TR caused by prolapse or "flail" of multiple flaps, as typically seen in post--traumatic TR and severe degenerative TR, cannot be corrected by a simple annuloplasty procedure.

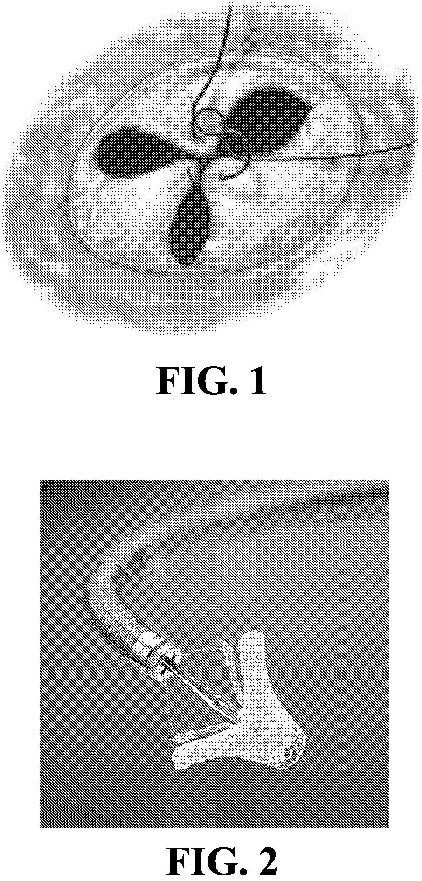

In order to obtain an effective and lasting repair, the so-called "clover" technique has been proposed. This technique consists of tying together the central part of the free edges of the tricuspid flaps, producing a valve in the shape of a "clover". A visual representation of a tricuspid valve treated according to this technique is shown in figure 1. Clinical results obtained with this technique are reported in:
- *"*The 'clover technique' as a novel approach for correction of post-traumatic tricuspid regurgitation", O. Alfieri, M. De Bonis et al., Journal of Thoracic and Cardiovascular Surgery, 2003; Vol. 126, No. 1, pages 75-79;
- *"*A novel technique for correction of severe tricuspid valve regurgitation due to complex lesions." De Bonis M, Lapenna E et al. Eur. J. Cardiothorac. Surg. 2004 May; 25 (5): 760-5.
- "Four-leaflet clover repair of severe tricuspid valve regurgitation due to complex lesions", E. Lapenna, M. De Bonis et al., Journal of Cardiovascular Medicine, 2008, Vo. 9 No. 8, pages 847-849;
- *"*The clover technique for the treatment of complex tricuspid valve insufficiency: midterm clinical and echocardiographic results in 66 patients", E. Lapenna, M. De Bonis et al., European Journal of Cardio-thoracic Surgery, 37 (2010), 1297 -1303;
- *"*Long-term results (up to 14 years) of the clover technique for the treatment of complex tricuspid valve regurgitation", De Bonis M, Lapenna E, et al. Eur. J. Cardiothorac. Surg. 2017 Feb. 23. doi: 10.1093 / ejcts / ezx027.

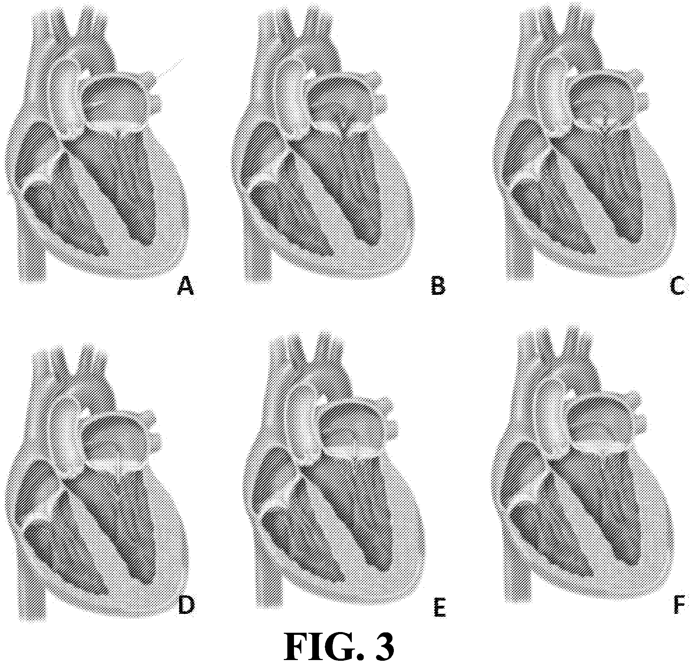

Devices for catching opposite flaps of a mitral valve as well as a tricuspid valve are sold under the trade names MITRACLIP^{™} and TRICLIP^{™}. This prior device, which can be introduced into the heart through a catheter in a blood vessel or through a small incision in the chest, comprises an applicator of a catching device of the type shown in figure 2. The sequence of operations to be performed to implant a device MITRACLIP^{™} catching device is shown in figure 3. In the illustrated case the heart valve is the mitral valve, but the same observations apply *mutatis mutandis* also for the tricuspid valve. Using a catheter, the MITRACLIP^{™} catching device is inserted in a folded configuration into the heart; when the catheter is close to the heart valve, the latch is deployed like an umbrella to catch the valve flaps, and is subsequently closed to hold the flaps together. Finally, the MITRACLIP^{™} catching device is left closed in the heart to hold the flaps together, thereby reducing valve regurgitation.

### SUMMARY

Unfortunately, tests performed by the Applicant have shown that this known applicator with two arms is unable to simultaneously catch all three flaps of the tricuspid valve, thus its effectiveness in treating tricuspid regurgitation is very limited. In the presence of a very dilated tricuspid annulus, even catching only two flaps of the tricuspid valve is quite difficult with the MITRACLIP^{™} device. When more MITRACLIP^{™} devices are implanted in order to improve the continence of the tricuspid valve, the risk of tricuspid stenosis increases significantly, greatly reducing the area of the repaired tricuspid valve. In order to prevent this problem, the device of the present disclosure is designed to simultaneously retain all three flaps of the tricuspid valve, or the two flaps of the mitral valve, so as to maintain them assuming a final configuration as in the common surgical procedure.

This exceptional result is achieved with a prosthesis as defined in the appended claim 1. The prosthesis is the portion, left in the patient's heart, of a related device for repairing a tricuspid or mitral valve, comprising a prosthesis of this disclosure inserted in a catheter for interventions.

Further embodiments are defined in the attached claims.

### BRIEF DESCRIPTION OF THE DRAFINS

Figure 1 schematically shows a typical configuration of a tricuspid valve after surgery with the so-called "clover" technique.
Figure 2 shows a known attachment device for flaps of a tricuspid or mitral valve.
Figure 3 shows various steps for implanting the so-called MITRACLIP^{™} catching device to the flaps of a heart valve.
Figure 4 shows a device according to an embodiment for repairing a tricuspid or mitral valve, comprising a prosthesis of this disclosure inserted into an outermost catheter, with the fins in a retracted configuration extended longitudinally along the respective tubular support.
Figure 5 shows the device of Figure 4 while the first three fins are released.
Figure 6 shows the device of Figure 5 with the first three fins deployed radially.
Figure 7 shows the device of Figure 6 with the second three fins deployed radially.
Figure 8 shows the device of Figure 7 when the second three fins are pushed towards the first three fins.
Figure 9 shows the device of Figure 8 with the first three fins pushed into the open cavity defined under the cap so that the first three fins are wedged and firmly locked between the cap and the third tubular support.
Figure 10 shows the prosthesis of the device of figure 9, after having released a first, a second and a third catheter from the respective tubular supports and after having removed a fourth outermost catheter.
Figure 11 shows the prosthesis of figure 10 tied to the respective first, second and third catheters, with the elastic stop finger which does not engage a tooth of a first rack of the snap lock.
Figure 12 shows the prosthesis of figure 11 tied to the respective first, second and third catheters to allow the repositioning of the fins, with the elastic stop finger which engages a tooth of the first rack of the snap lock on the second tubular support.
Figure 13 is a detail view of the prosthesis in figure 11, shofin the elastic stop finger raised from the first rack through the release ring.
Figure 14 is a detail view of the prosthesis in Figure. 12, shofin the elastic stop finger which engages a tooth of the first rack.
Figure 15 shows a device according to an alternative embodiment for repairing a tricuspid or mitral valve, comprising a prosthesis of this disclosure inserted into an outermost catheter, with the fins in a retracted configuration extended longitudinally along the respective tubular support.
Figure 16 shows the device of Figure 15 while the first three fins are released.
Figure 17 shows the device of Figure 16 with the first three fins deployed radially.
Figure 18 shows the device of Figure 17 with the second three fins deployed radially.
Figure 19 shows the device of Figure 18 when the second three fins are pushed towards the first three fins.
Figure 20 shows the device of figure 19 with the first three fins pushed into the open cavity defined under the cap so that the first three fins are wedged and firmly locked between the cap and the third tubular support.
Figure 21 shows the prosthesis of the device of figure 20, after having released a first, a second and a third catheter from the respective tubular supports and after having removed a fourth outermost catheter.
Figures 22 to 24 show in sequence how to hook the second tubular support 3 to the cap by engaging the first rack with the second rack defined on an internal wall of the cap.

### DETAILED DESCRIPTION OF EXAMPLE EMBODIMENTS

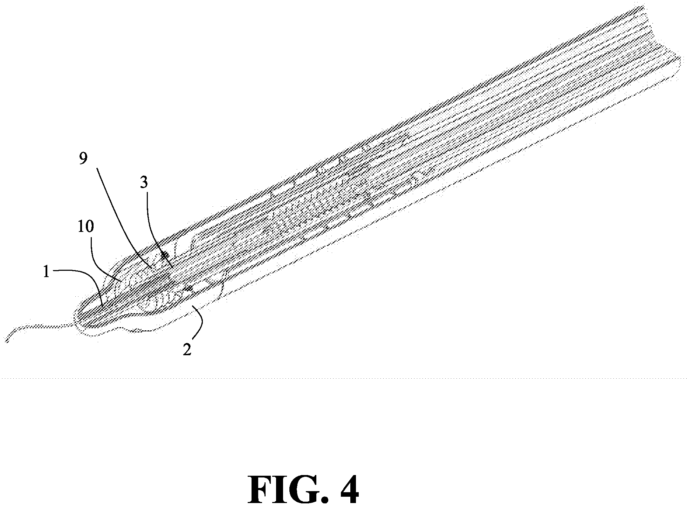

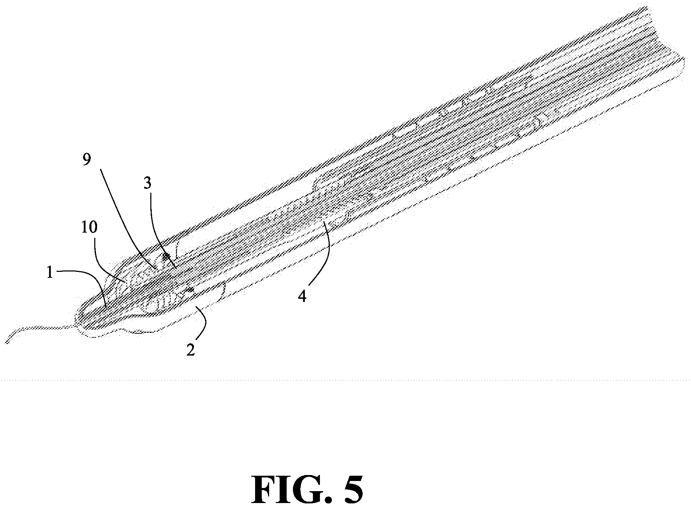

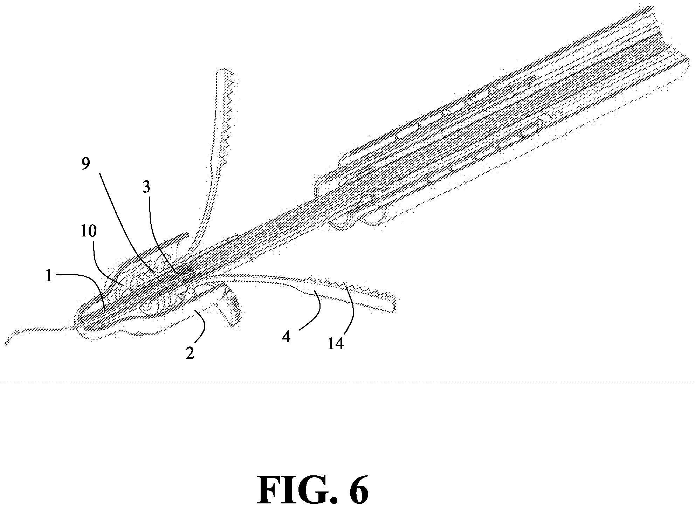

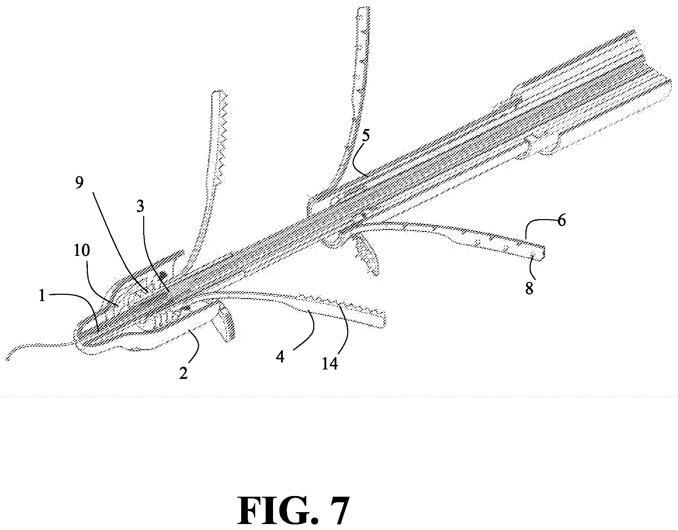

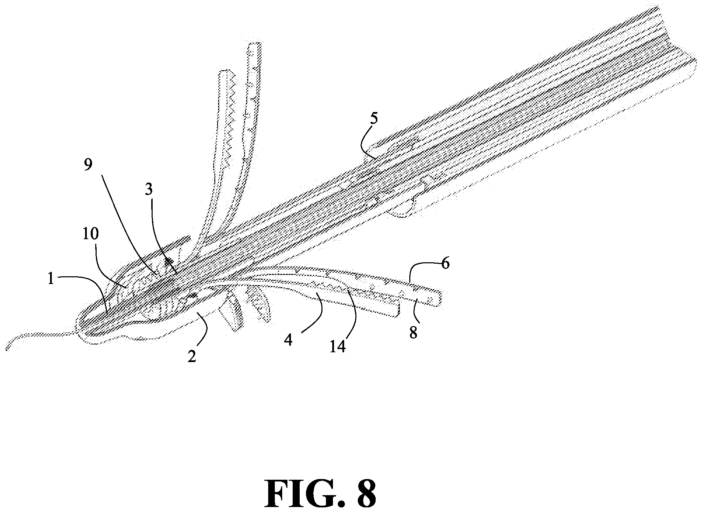

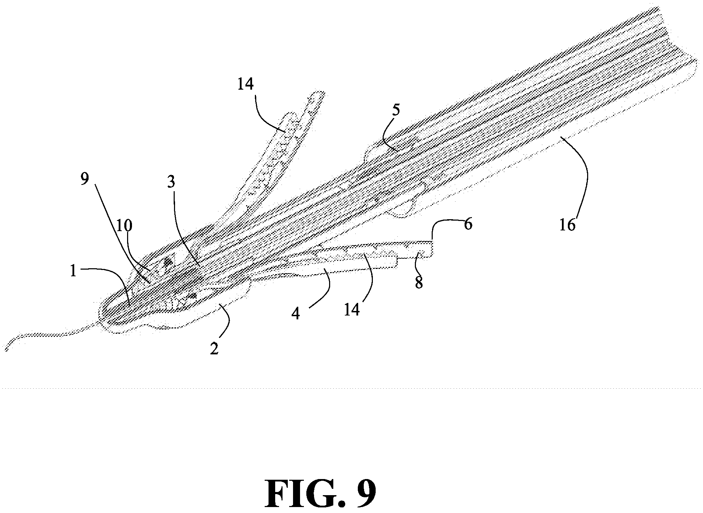

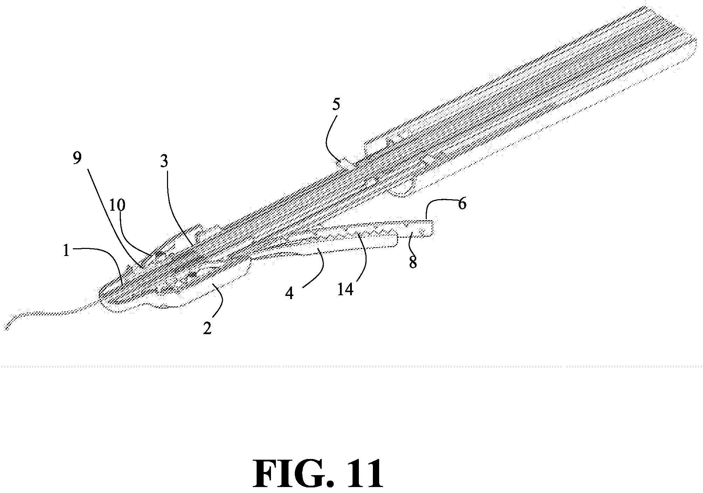

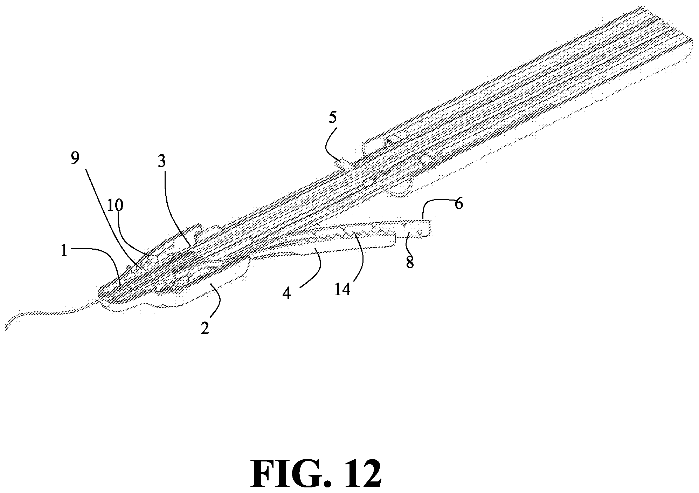

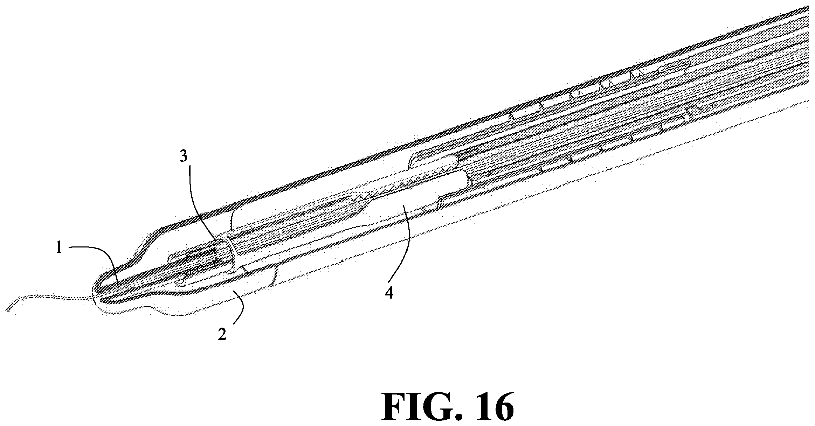

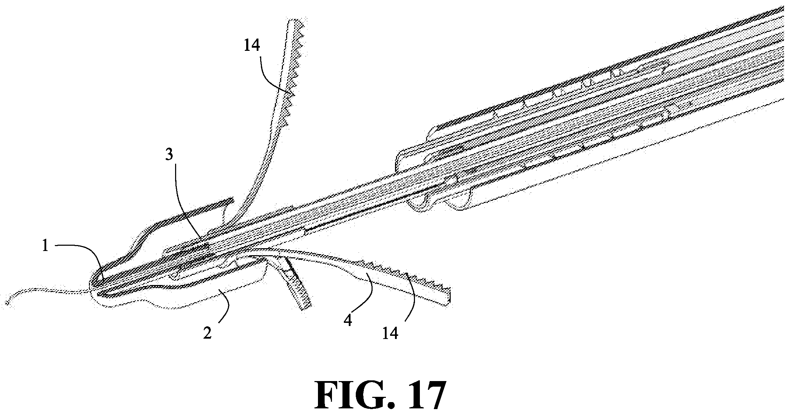

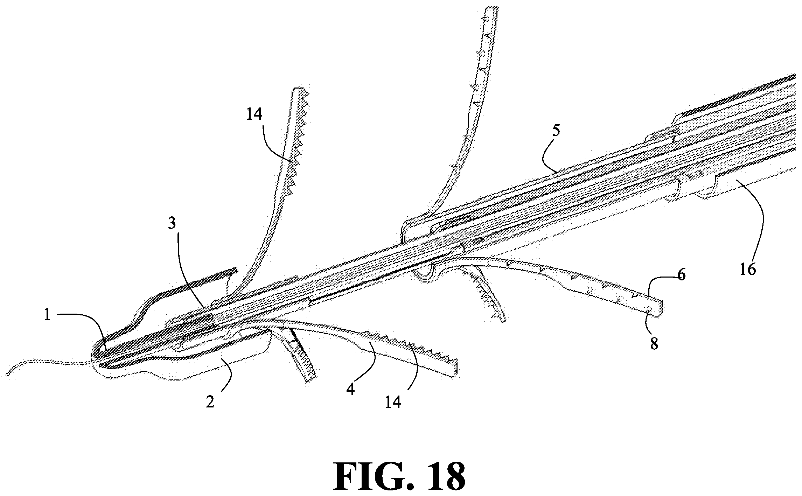

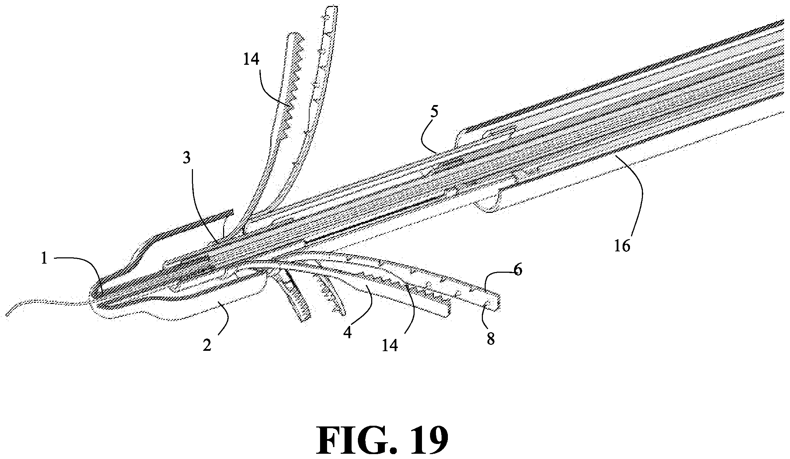

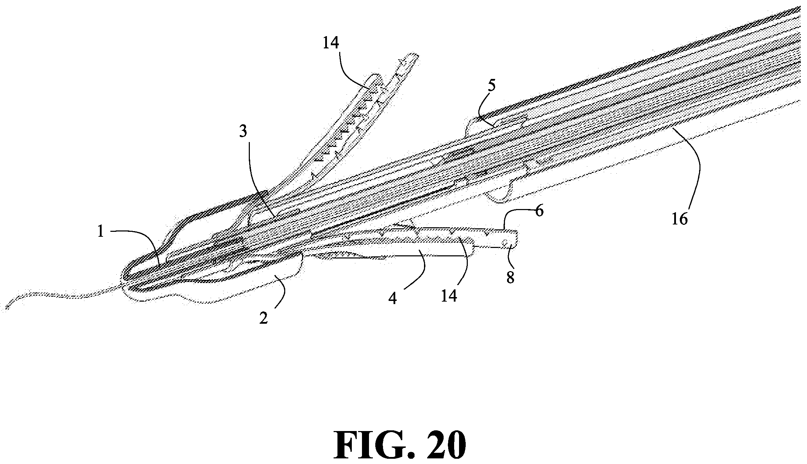

Prostheses of this disclosure for repairing a tricuspid or mitral valve are schematically illustrated in Figures 4 to 24.

In the follofin description reference will be made to the repair of a tricuspid valve, but the same observations apply *mutatis mutandis* to the repair of a mitral valve. For this reason, the prosthesis shown in the figures has angularly spaced triples of fins for catching the three flaps of a tricuspid valve. However, the prosthesis shown could be made with two pairs of fins or with two groups with more than three fins each for catching the flaps of any heart valve. Furthermore, it is not essential that the triplets of fins are arranged with radial symmetry, as exemplified in the figure: the fins may be angularly spaced one from the other in various ways to adapt to the anatomy of the patients' heart valves.

In one aspect, a prosthesis for a tricuspid or mitral valve of this disclosure comprises a first tubular support 1 having a first tubular body, terminating at its distal end with an cap 2 open like an umbrella. The cap 2 defines, together with the outer wall of the first tubular support 1, a cavity open on one side.

Optionally, the first tubular support 1 has a longitudinal axial hole through which to pass a guide wire, of the type commonly used in surgery to position heart valves to be implanted.

The prosthesis of this disclosure also has a second tubular support 3 having a second tubular body with a respective longitudinal axial hole, with a first plurality of fins 4 constrained to a distal portion of the second tubular support 3, and a third tubular support 5, having a third tubular body with a respective longitudinal axial hole, having at least a second plurality of fins 6 constrained to a distal portion of the third tubular support 5. The fins of the first plurality of fins 4 and of the second plurality of fins 6 are configured to pass autonomously from a respective retracted configuration, in which they are extended longitudinally along an external wall respectively of the second tubular support 3 and of the third tubular support 5, to a respective open configuration, in which they are open radially away from the external wall of the second tubular support 3 and of the third tubular support 5, respectively, as soon as they are free to unfold.

In the exemplary embodiment shown in Figures 4 to 14, each plurality of fins 4 and 6 is formed by three fins, because the prosthesis shown is made for tricuspid valves. Conveniently, the first plurality of fins 4 comprises three first fins angularly spaced by 120°, and the second plurality of fins 6 comprises three second fins angularly spaced by 120° placed in front of the first plurality.

As already mentioned, it is not essential that the fins are regularly spaced by 120°. It is possible to space the fins unevenly from each other to adapt to the anatomy of the patient's heart valve. Furthermore, it is not essential to have fins of the same shape and size, as shown in the attached drawings, but it will be possible for example to make a first plurality of fins 4 wider or longer than the second plurality of fins 6 or vice versa.

However, it is possible to make each plurality of fins with two or four or even more fins to better catch the flaps of the heart valves.

The second tubular support is configured to slide in a longitudinally guided manner over the outer wall of and concentrically to the body of the first tubular support 1, just as the third tubular support 5 is configured to slide in a longitudinally guided manner over the outer wall of and concentrically to the body of the second tubular support 3 and above the first plurality of fins 4 in a retracted configuration.

The fins of the first plurality of fins 4 and of the second plurality of fins 6 have respective facing surfaces 7 and 8 configured to cooperate with each other to catch a flap of a tricuspid or mitral valve by squeezing it like a jaw from opposite faces. According to an optional aspect, the respective facing surfaces 7 and 8 of the fins of the first plurality of fins 4 and of the second plurality of fins 6, respectively, have teeth and / or harpoons 14 for catching the flaps of a tricuspid or mitral valve. In the example shown in the figures, the fins 4 have a knurling while the fins 6 have harpoons, but nothing prevents from having a different configuration from the one shown. For example, to have reversible catching mechanisms, fins 6 could be made which have harpoons or spikes which engage in corresponding concavities made on the fins 4. Alternatively, fins 4 and 6 could be made, both with a knurling or with a significant surface roughness whereby, by pressing the fins 4 and 6 so as to pinch a flap of a heart valve, this is firmly held by friction.

As shown in the succession of figures, the second tubular support 3 is pushed so as to free the fins 4 of the first plurality of fins. As soon as they are outside the third tubular support 5, they extend radially by themselves opening like "petals". In the embodiment shown in figures 4 to 14, the third tubular support 7 is pushed so as to free the fins 6 of the second plurality of fins. Like the fins 4 of the first plurality, the fins 6 also extend radially by themselves.

The automatic opening of the fins 4 and 6 may be conveniently implemented by making the fins 4 and 6 in a shape memory material, such as Nitinol. Alternatively, the fins 4 and 6 could be made of elastic material so that as soon as they come out of the third tubular support 5, they tend to elastically open. In this way, the fins 4 and 6 will remain extended along the second tubular support 3 and the third tubular support 5 until the prosthesis is in the closed configuration of figure 4, in which it will be distributed, but they will open to catch the flaps of a valve heart as soon as they are free to move.

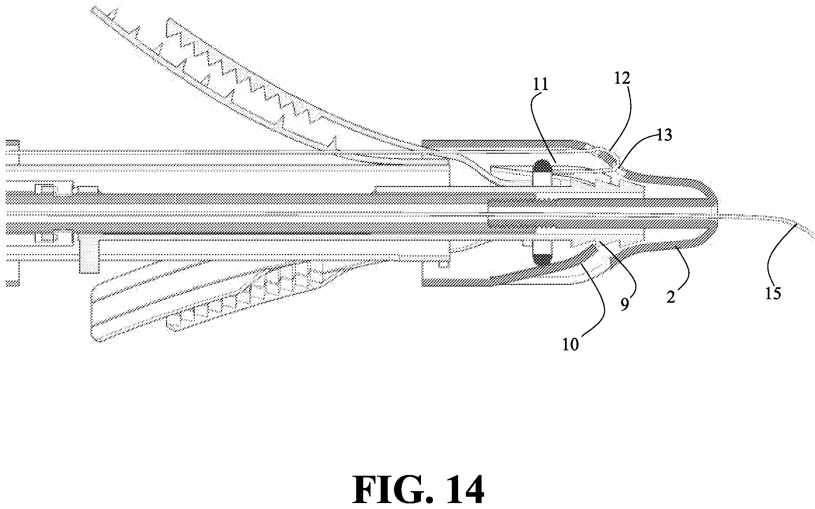

In order to catch the flaps of a valve, the second tubular support 3 and the third tubular support 5 are pushed so as to bring the fins 4 of the first plurality closer to the corresponding fins 6 of the second plurality and so as to insert the distal portion of the second tubular support 3, to which the fins 4 of the first plurality of fins are fixed, inside the cavity defined by the cap 2 with the first tubular support 1. By forcefully pushing the second 3 and third 5 tubular supports into the cavity, the fins 4 of the first plurality remain wedged between the edge of the cap 2 and the third tubular support 5, so that a flap of a heart valve sandwiched between the fins 4 and 6 will be firmly held in place. In the embodiment illustrated in figures 4 to 14, the outer wall of the second tubular support 3 defines a rack 9 at the distal portion for locking the fins 4 and 6 in position once a flap of a heart valve has been catched. The cap 2 open like an umbrella has an elastic stop finger 10 that protrudes inside the cavity, and together with the rack 9 forms a snap-lock device. The elastic finger 10 is configured to engage at least one tooth of the rack 9 by sliding longitudinally above it when the distal portion of the second tubular support 3 is pushed into the cavity. As can be seen more clearly in the detail view of figure 14, the elastic finger 10 abuts against a tooth of the rack 9 and prevents the distal portion of the second tubular support 3 from accidentally exiting the cavity.

Preferably, the rack 9 will have a plurality of teeth, as illustrated in the figure, so as to allow a fine adjustment of the force with which the flaps of the valve are held. In fact, by pulling the cap 2 with gradually increasing force, the elastic finger 10 is made to slide which, step by step, will engage the teeth of the rack 9 in succession, establishing different stop positions of the cap 2. The cap 2, while advancing, tends to bring the fins 4 and 6 closer together, which thus tighten a respective flap of a valve together. By adjusting the force with which the cap 2 is pulled, the force with which the fins 4 and 6 tighten the relative valve flap as well as the angle of the fins 4 and 6 themselves are also determined. Thanks to this mechanism, the surgeon will be able to decide how strongly the valve flaps must be held, preventing fins 4 and 6 from tearing the relatively thick flaps or letting the relatively thin flaps slip out, simply by establishing how far the cap 2 should advance. Once this has been done, the elastic finger 10 abutting against a relative tooth of the rack 9 will keep the cap 2 in position.

Pushing the distal portion of the second tubular support 3 into the cavity defined by the cap 2 could require considerable effort. To circumvent this drawback, according to an optional aspect of the prosthesis of the present disclosure, the cap 2 is screwed onto the first tubular support 1, as shown in the figures. Thanks to this expedient, it is sufficient to rotate the first tubular support 1 so as to pull the cap 2 towards the second tubular support 3, until the elastic finger 10 abuts on a respective tooth of the rack 9, blocking the second tubular support 3 in that position (figure 14).

According to one aspect, the cap 2 may be made of rigid material. According to an alternative aspect, the cap 2 may be made of an elastic material so as to make the thrust of the second tubular support 3 into the cavity defined by the cap 2 more gradual. According to an aspect not shown in the drawings, the free edge of the cap 2 is shaped in such a way as to define fins that branch off from it like petals of a corolla.

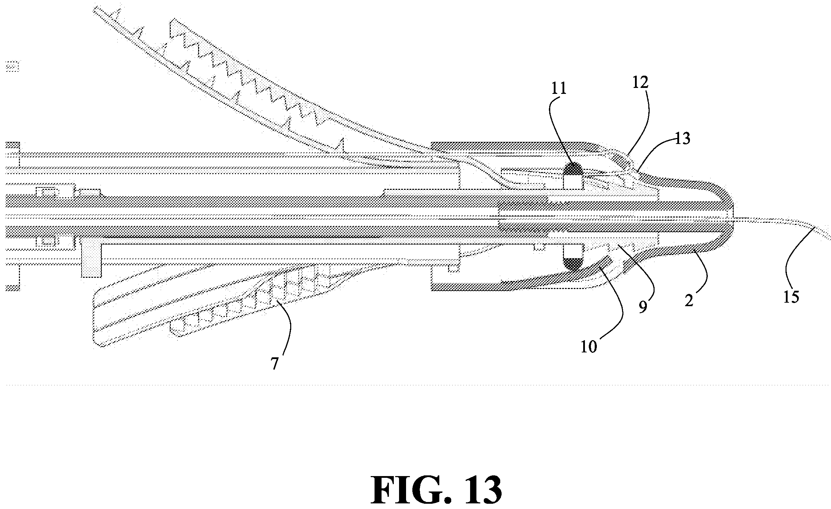

When placing the prosthesis, it may be necessary to re-space the fins 4 and 6 to better catch the flaps of a heart valve, because perhaps a flap has slipped while locking the second tubular support 3 in the cavity of the cap 2. For this reason, according to an optional aspect, the prosthesis of this disclosure also has a release ring 11 positioned around the second tubular support 3 so as to slide over it, as well as a release wire 12 fixed to the release ring 11, which passes through at least one through hole 13 in the cap 2 and runs longitudinally along the outer wall of the third tubular support 5. The release wire 12 and the release ring 12 are configured so that, by pulling the release wire 12 in the manner shown in figure 13, the release ring 11 moves longitudinally entering the cavity defined by the cap 2 by lifting the elastic finger 10. Consequently, the elastic finger 10 of the engagement will no longer abut against a tooth of the rack 9 and will allow the distal portion of the second tubular support 3 to come out of the cavity. The fins 4 and 6 may then be repositioned to better catch the flaps of a heart valve, after which they will be stopped in that configuration by pushing them longitudinally towards the cavity defined by the cap 2 until the elastic stop finger 10 engages a tooth of the rack 9.

According to an optional aspect, a surgical guide wire 15 is inserted into the longitudinal axial hole of the first tubular support 1, so as to be able to guide the prosthesis in the center of the heart valve.

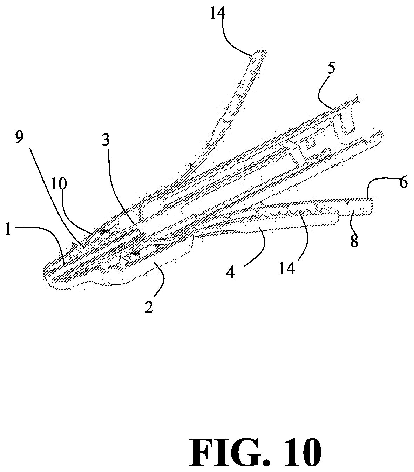

In order to place the prosthesis of this disclosure, shown in Figure 10, the device of this disclosure comprises a first catheter, a second catheter and a third catheter releasably connected to the first tubular support 1, the second tubular support 3 and the third tubular support 5, respectively. Through the second and third catheters it is possible to move longitudinally and rotate the fins 4 and 6, while with the first catheter it is possible to pull the cap 2 towards the fins 4 and 6. Once the fins 4 and 6 tighten the flaps of a heart valve and the elastic finger 10 engages a tooth of the rack 9, the first, second and third catheters are released and the prosthesis (Figure 10) is left in the patient's body.

According to an aspect not shown in the drawings, the first tubular support 1, the second tubular support 3 and the third tubular support 5 are integral with the corresponding catheters, respectively the first catheter, the second catheter and the third catheter, and constitute an extension thereof. Once the prosthesis is correctly installed in the patient's body, the tubular supports are separated from their respective catheters by a cutting operation. In this way, the length of the tubular supports that are left in the patient's body is determined by the surgeon as needed by establishing the point in which to cut the respective catheters.

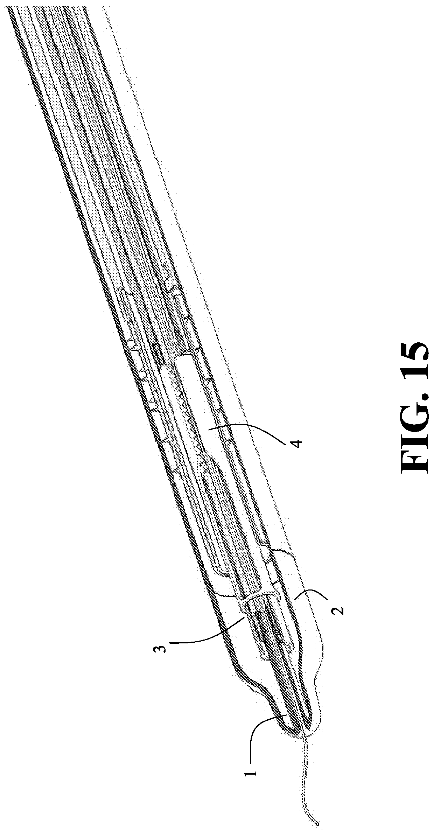

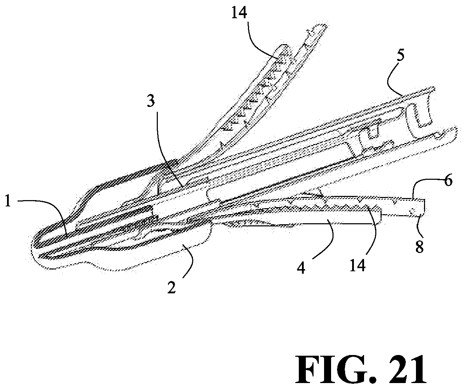

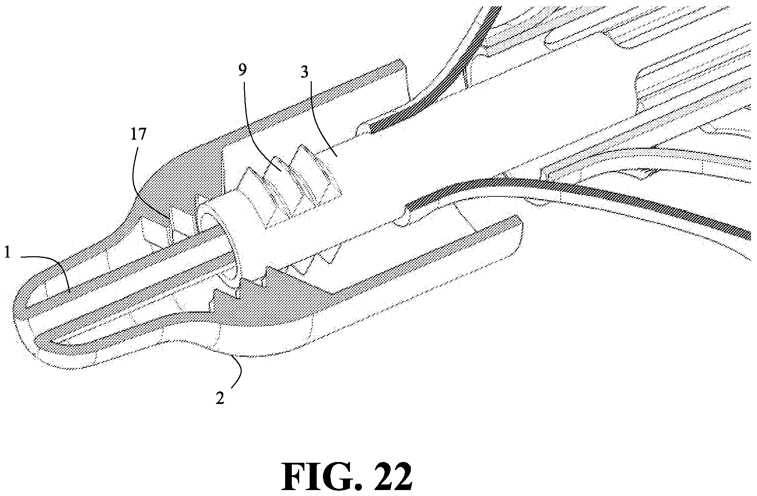

An alternative embodiment of a prosthesis according to this disclosure is illustrated in figures 15 to 24, in which the elements corresponding to those illustrated in figures 4 to 14 are designated with the same numerical references. Figures 15 to 20 are similar to figures 4 to 9 and show how the fins 4 and 6 unfold and tighten so as to pinch the flaps of a heart valve, and figure 21 is similar to figure 10 and shows the prosthesis which is implanted in the patient's body. The prosthesis according to this alternative embodiment has a longitudinal sliding stop device illustrated in figures 22 to 24. As for the embodiment shown in figures 13 and 14, the outer wall of the second tubular support 3 defines in correspondence of its distal portion a first rack 9 intended, as in figures 13 and 14, to engage corresponding mechanical stop defined on the cap 2.

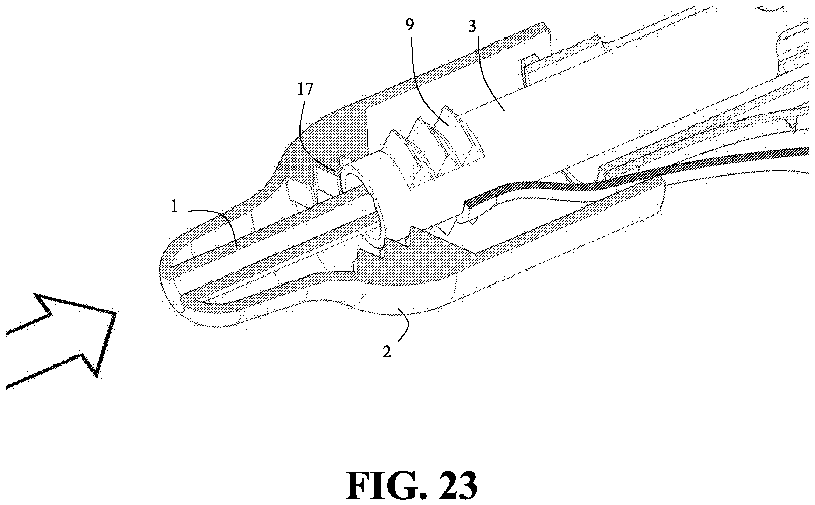

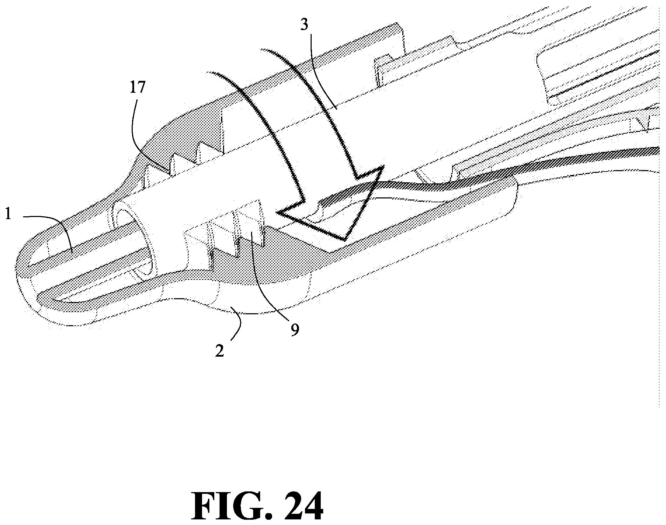

Unlike figures 13 and 14, in which the mechanical stop are substantially constituted by an elastic finger 10, in the embodiment of figures 22 to 24 the mechanical stop comprise a second rack 17 defined on an internal face of the cap 2 and configured to engage the first rack 9. As shown in the sectional views of figures 22 and 23, the second tubular support is pushed so that the cap 2 tightens the fins 4 and 6 against each other by sliding longitudinally the first rack 9 inside the cap 2. Once the cap 2 has been positioned so as to tighten the fins 4 and 6 with the desired force, the second tubular support 3 is rotated so that the teeth of the first rack 9 engage respective teeth of the second rack 17 inside the cap 2, as shown in figure 24, preventing the second tubular support 3 from accidentally slipping off.

If it is necessary to reposition the prosthesis, for example because the heart valve flaps have not been properly catched, the second tubular support 3 is rotated so as to free the first rack 9 from the second rack 17, then the second tubular support 3 is slid so as to free the fins 4 and 6 which, once repositioned, can be locked in the manner indicated above. As for the embodiment of figures 4 to 14, once the fins 4 and 6 tighten the flaps of a heart valve and the first rack 9 is locked, this time thanks to respective teeth of the second rack 17, all the first, second and third catheters are released and the prosthesis (figure 21) is left in the patient's body.

An outermost catheter 16, configured to slide longitudinally over the outer wall of the third tubular support 5, keeps the fins of the second plurality of fins 6 in the respective retracted configuration. Optionally, the fourth catheter 16 is configured to abut against a free edge of the cap 2.

The outermost catheter 16 is adapted to be inserted into a patient's heart through a vein, as shown in figure 3 and commonly done for the well-known MITRACLIP^{™} device, or through an incision in the patient's chest.

The prosthesis will be made of biocompatible materials suitable for heart prostheses to be implanted in the patient's heart.

## Claims

1. A prosthesis for a tricuspid or mitral valve, comprising:
a first tubular support (1) having a first tubular body with a respective longitudinal axial hole, terminating at a distal end with a cap (2) open like an umbrella around said first tubular support (1), wherein a cavity open on one side is defined between said cap (2) open like an umbrella and an outer wall of said first tubular support (1);
a second tubular support (3) having a second tubular body with a respective longitudinal axial hole, said second tubular body being configured to slide in a longitudinally guided manner over an external wall of and concentrically to said first tubular body of the first tubular support (1), having at least a first plurality of fins (4) constrained to a distal portion of the second tubular body of the second tubular support (3), the fins of said first plurality of fins (4) being configured to pass autonomously from a respective retracted configuration, in which they are extended longitudinally along an external wall of said second tubular body of the second tubular support (3), to a respective open configuration, in which they are open radially away from the external wall of the second tubular body of the second tubular support (3), as soon as they are free to unfold;
a third tubular support (5) having a third tubular body with a respective longitudinal axial hole, configured to slide in a longitudinally guided manner above and concentrically to said second tubular body of the second tubular support (3) and above said first plurality of fins (4) in the respective retracted configuration, having at least a second plurality of fins (6) tied to a distal portion of the third tubular support (3), the fins of said second plurality of fins (6) being configured to pass autonomously from a respective retracted configuration, in which they are extended longitudinally along an external wall of said third tubular support (5), to a respective open configuration, in which they are open radially away from the external wall of the third tubular support (5), as soon as they are free to unfold;
wherein the fins of said first plurality of fins (4) and of said second plurality of fins (6) have respective facing surfaces (7, 8) configured to cooperate with each other to catch a flap of a tricuspid or mitral valve by squeezing it from opposite faces;
the outer wall of said second tubular support (3) defines, in correspondence with said distal portion, a first rack (9) of a locking device with longitudinal sliding stop;
said cap open like an umbrella having a mechanical stop, configured to prevent longitudinal sliding of the second tubular support (3) when said mechanical stop engage said first rack (9).

2. The prosthesis according to claim 1, wherein said locking device with longitudinal sliding stop is a snap lock and said umbrella-like open cap (2), having said mechanical stop, has an elastic stop finger (10), which protrudes inside said cavity and is configured to engage at least one tooth of said first rack (9) by sliding above it when the distal portion of the second tubular support (3) is pushed into said cavity by sliding above said first tubular support (1), so as to prevent the distal portion of the second tubular support (3) from accidentally coming out of said cavity.

3. The prosthesis of claim 2, comprising:
an release ring (11) positioned around said second tubular support (3) so as to slide over it;
a release wire (12) fixed to the release ring (11), which passes through at least one through hole (13) in the cap (2) and runs longitudinally along said external wall of the third tubular support (5);
said release wire (12) and said release ring (12) being configured so that, by pulling the release wire (12), the release ring (11) moves longitudinally entering the cavity defined by the cap (2) and lifting the elastic stop finger (10) from said first rack (9) allofin the distal portion of the second tubular support (3) to exit from said cavity.

4. The prosthesis according to claim 1, wherein said cap (2) open like an umbrella, having said mechanical stop, has a second rack (17) defined on an internal face of the cap (2) and configured to engage the first rack (9) by axially rotating said second tubular support (3) axially on itself with respect to said cap (2).

5. The prosthesis according to one of the preceding claims, in which said cap (2) is screwed onto said first tubular support (1).

6. The prosthesis according to one of the preceding claims, in which said first fins (14) and said second fins (6) are made of Nitinol and are configured to unfold radially as soon as they are free to unfold.

7. The prosthesis according to one of the preceding claims, in which said respective facing surfaces (7, 8) of said fins (4, 6) have teeth and / or harpoons (14) for catching flaps of a tricuspid or mitral valve.

8. The prosthesis according to one of the preceding claims, further comprising a surgical guide wire (15) inserted into the longitudinal axial hole of the first tubular support (1).

9. The prosthesis according to one of the preceding claims, wherein said first plurality of fins (4) comprises three first fins angularly spaced by 120°, and wherein said second plurality of fins (6) comprises three second fins angularly spaced by 120°.

10. A device for repairing a tricuspid or mitral valve, comprising:
a prosthesis according to one of the preceding claims;
a first catheter releasably connected to said first tubular support (1);
a second catheter releasably connected to said second tubular support (3);
a third catheter releasably connected to said third tubular support (5);
a fourth catheter (16), configured to slide longitudinally over the outer wall of said third tubular support (5) and over the fins of said second plurality of fins (6) in the respective retracted configuration.

11. The device according to the preceding claim, wherein said fourth catheter (16) is configured to abut against a free edge of said cap (2) open like an umbrella of the prosthesis.

## Patentansprüche

1. Prothese für eine Trikuspidal- oder eine Mitralklappe, umfassend:
einen ersten rohrförmigen Träger (1), der einen ersten rohrförmigen Körper mit einem entsprechenden axialen Längsloch aufweist, der an einem distalen Ende mit einer Kappe (2) endet, die schirmartig um den ersten rohrförmigen Träger (1) offen ist, wobei ein Hohlraum, der auf einer Seite offen ist, zwischen der Kappe (2), die schirmartig offen ist, und einer Außenwand des ersten rohrförmigen Trägers (1) definiert ist;
einen zweiten rohrförmigen Träger (3), der einen zweiten rohrförmigen Körper mit einem entsprechenden axialen Längsloch aufweist, wobei der zweite rohrförmige Körper konfiguriert ist, um in einer längsgeführten Weise über eine Außenwand des ersten rohrförmigen Körpers des ersten rohrförmigen Trägers (1) und konzentrisch zu diesem zu gleiten, aufweisend mindestens eine erste Vielzahl von Finnen (4), die an einem distalen Abschnitt des zweiten rohrförmigen Körpers des zweiten rohrförmigen Trägers (3) festgemacht sind, wobei die Finnen der ersten Vielzahl von Finnen (4) konfiguriert sind, um von einer entsprechenden eingefahrenen Konfiguration, in der sie sich in Längsrichtung entlang einer Außenwand des zweiten rohrförmigen Körpers des zweiten rohrförmigen Trägers (3) erstrecken, in eine entsprechende offene Konfiguration selbstständig überzugehen, in der sie radial weg von der Außenwand des zweiten rohrförmigen Körpers des zweiten rohrförmigen Trägers (3) geöffnet sind, sobald sie sich frei entfalten können;
einen dritten rohrförmigen Träger (5), der einen dritten rohrförmigen Körper mit einem entsprechenden axialen Längsloch aufweist, der konfiguriert ist, um in einer längsgeführten Weise über und konzentrisch zu dem zweiten rohrförmigen Körper des zweiten rohrförmigen Trägers (3) und über die erste Vielzahl von Finnen (4) in der entsprechenden eingefahrenen Konfiguration zu gleiten, mindestens aufweisend eine zweite Vielzahl von Finnen (6), die an einem distalen Abschnitt des dritten rohrförmigen Trägers (3) befestigt sind, wobei die Finnen der zweiten Vielzahl von Finnen (6) konfiguriert sind, um von einer entsprechenden eingefahrenen Konfiguration, in der sie sich in Längsrichtung entlang einer Außenwand des dritten rohrförmigen Trägers (5) erstrecken, in eine entsprechende offene Konfiguration selbständig überzugehen, in der sie radial weg von der Außenwand des dritten rohrförmigen Trägers (5) geöffnet sind, sobald sie sich frei entfalten können;
wobei die Finnen der ersten Vielzahl von Finnen (4) und der zweiten Vielzahl von Finnen (6) entsprechende einander zugewandte Oberflächen (7, 8) aufweisen, die konfiguriert sind, um miteinander zusammenzuwirken, um ein Segel einer Trikuspidal- oder Mitralklappe zu fassen, indem sie diese von gegenüberliegenden Flächen zusammendrücken;
die Außenwand des zweiten rohrförmigen Trägers (3), in Übereinstimmung mit dem distalen Abschnitt, eine erste Zahnstange (9) einer Verriegelungsvorrichtung mit Längsgleitanschlag definiert;
wobei die Kappe, die schirmartig offen ist, einen mechanischen Anschlag aufweist, der konfiguriert ist, um ein Längsgleiten des zweiten rohrförmigen Trägers (3) zu verhindern, wenn der mechanische Anschlag in die erste Zahnstange (9) eingreift.

2. Prothese nach Anspruch 1, wobei die Verriegelungsvorrichtung mit Längsgleitanschlag ein Schnappverschluss ist und die schirmartige offene Kappe (2), die den mechanischen Anschlag aufweist, einen elastischen Anschlagfinger (10) aufweist, der in das Innere des Hohlraums hineinragt und konfiguriert ist, um in mindestens einen Zahn der ersten Zahnstange (9) einzugreifen, indem er darüber gleitet, wenn der distale Abschnitt des zweiten rohrförmigen Trägers (3) in den Hohlraum geschoben wird, indem er über den ersten rohrförmigen Träger (1) gleitet, um zu verhindern, dass der distale Abschnitt des zweiten rohrförmigen Trägers (3) versehentlich aus dem Hohlraum herauskommt.

3. Prothese nach Anspruch 2, umfassend:
einen Auslösering (11), der um den zweiten rohrförmigen Träger (3) angeordnet ist, um darüber zu gleiten;
einen Auslösedraht (12), der an dem Auslösering (11) befestigt ist, durch mindestens ein Durchgangsloch (13) in der Kappe (2) verläuft und in Längsrichtung entlang der Außenwand des dritten rohrförmigen Trägers (5) läuft;
wobei der Auslösedraht (12) und der Auslösering (12) konfiguriert sind, sodass, durch Ziehen an dem Auslösedraht (12), sich der Auslösering (11) in Längsrichtung bewegt, in den Hohlraum, der durch die Kappe (2) definiert ist, eintritt und den elastischen Anschlagfinger (10) von der ersten Zahnstange (9) abhebt, wobei dem distalen Abschnitt des zweiten rohrförmigen Trägers (3) ermöglicht wird, aus dem Hohlraum auszutreten.

4. Prothese nach Anspruch 1, wobei die Kappe (2), die schirmartig offen ist, die den mechanischen Anschlag aufweist, eine zweite Zahnstange (17) aufweist, die an einer Innenfläche der Kappe (2) definiert und konfiguriert ist, um in die erste Zahnstange (9) einzugreifen, durch axiales Drehen des zweiten rohrförmigen Trägers (3) axial um sich selbst in Bezug auf die Kappe (2).

5. Prothese nach einem der vorstehenden Ansprüche, wobei die Kappe (2) auf den ersten rohrförmigen Träger (1) aufgeschraubt ist.

6. Prothese nach einem der vorstehenden Ansprüche, wobei die ersten Finnen (4) und die zweiten Finnen (6) aus Nitinol gefertigt sind und konfiguriert sind, um sich radial zu entfalten, sobald sie sich frei entfalten können.

7. Prothese nach einem der vorstehenden Ansprüche, wobei die entsprechenden einander zugewandten Flächen (7, 8) der Finnen (4, 6) Zähne und/oder Sicherungsklinken (14) zum Fassen von Segeln einer Trikuspidal- oder Mitralklappe aufweisen.

8. Prothese nach einem der vorstehenden Ansprüche, ferner umfassend einen chirurgischen Führungsdraht (15), der in das axiale Längsloch des ersten rohrförmigen Trägers (1) eingeführt ist.

9. Prothese nach einem der vorstehenden Ansprüche, wobei die erste Vielzahl von Finnen (4) drei erste Finnen in einem Winkelabstand von 120° umfasst, und wobei die zweite Vielzahl von Finnen (6) drei zweite Finnen in einem Winkelabstand von 120° umfasst.

10. Vorrichtung zum Reparieren einer Trikuspidal- oder Mitralklappe, umfassend:
eine Prothese nach einem der vorstehenden Ansprüche;
einen ersten Katheter, der mit dem ersten rohrförmigen Träger (1) lösbar verbunden ist;
einen zweiten Katheter, der mit dem zweiten rohrförmigen Träger (3) lösbar verbunden ist;
einen dritten Katheter, der mit dem dritten rohrförmigen Träger (5) lösbar verbunden ist;
einen vierten Katheter (16), der konfiguriert ist, um in Längsrichtung über die Außenwand des dritten rohrförmigen Trägers (5) und über die Finnen der zweiten Vielzahl von Finnen (6) in der entsprechenden eingefahrenen Konfiguration zu gleiten.

11. Vorrichtung nach dem vorstehenden Anspruch, wobei der vierte Katheter (16) konfiguriert ist, um an einem freien Rand der Kappe (2) der Prothese, die schirmartig offen ist, anzuliegen.

## Revendications

1. Prothèse pour une valve tricuspide ou mitrale, comprenant :
un premier support tubulaire (1) ayant un premier corps tubulaire avec un trou axial longitudinal respectif, se terminant au niveau d'une extrémité distale par un capuchon (2) ouvert comme un parapluie autour dudit premier support tubulaire (1), dans laquelle une cavité ouverte sur un côté est définie entre ledit capuchon (2) ouvert comme un parapluie et une paroi extérieure dudit premier support tubulaire (1) ;
un deuxième support tubulaire (3) ayant un deuxième corps tubulaire avec un trou axial longitudinal respectif, ledit deuxième corps tubulaire étant conçu pour coulisser de manière guidée longitudinalement par-dessus une paroi externe dudit premier corps tubulaire du premier support tubulaire (1) et concentriquement à celui-ci, ayant au moins une première pluralité d'ailettes (4) contraintes à une partie distale du deuxième corps tubulaire du deuxième support tubulaire (3), les ailettes de ladite première pluralité d'ailettes (4) étant conçues pour passer de manière autonome d'une configuration rétractée respective, dans laquelle elles sont étendues longitudinalement le long d'une paroi externe dudit deuxième corps tubulaire du deuxième support tubulaire (3), à une configuration ouverte respective, dans laquelle elles sont ouvertes radialement à distance de la paroi externe du deuxième corps tubulaire du deuxième support tubulaire (3), dès qu'elles sont libres de se déployer ;
un troisième support tubulaire (5) ayant un troisième corps tubulaire avec un trou axial longitudinal respectif, conçu pour coulisser de manière guidée longitudinalement au-dessus dudit deuxième corps tubulaire du deuxième support tubulaire (3) et concentriquement à celui-ci et au-dessus de ladite première pluralité d'ailettes (4) dans la configuration rétractée respective, ayant au moins une seconde pluralité d'ailettes (6) attachées à une partie distale du troisième support tubulaire (3), les ailettes de ladite seconde pluralité d'ailettes (6) étant conçues pour passer de manière autonome d'une configuration rétractée respective, dans laquelle elles sont étendues longitudinalement le long d'une paroi externe dudit troisième support tubulaire (5), à une configuration ouverte respective, dans laquelle elles sont ouvertes radialement à distance de la paroi externe du troisième support tubulaire (5), dès qu'elles sont libres de se déployer ;
dans laquelle les ailettes de ladite première pluralité d'ailettes (4) et de ladite seconde pluralité d'ailettes (6) ont des surfaces en regard respectives (7, 8) conçues pour coopérer les unes avec les autres afin d'attraper un clapet d'une valve tricuspide ou mitrale en le pressant à partir de faces opposées ;
la paroi extérieure dudit deuxième support tubulaire (3) définit, en correspondance avec ladite partie distale, une première crémaillère (9) d'un dispositif de verrouillage avec une butée coulissante longitudinale ;
ledit capuchon ouvert comme un parapluie ayant une butée mécanique, conçue pour empêcher le coulissement longitudinal du deuxième support tubulaire (3) lorsque ladite butée mécanique vient en prise avec ladite première crémaillère (9).

2. Prothèse selon la revendication 1, dans laquelle ledit dispositif de verrouillage avec butée coulissante longitudinale est un verrou à encliquetage et ledit capuchon ouvert en forme de parapluie (2), ayant ladite butée mécanique, a un doigt de butée élastique (10), qui fait saillie à l'intérieur de ladite cavité et est conçu pour venir en prise avec au moins une dent de ladite première crémaillère (9) en coulissant au-dessus de celle-ci lorsque la partie distale du deuxième support tubulaire (3) est poussée dans ladite cavité par coulissement au-dessus dudit premier support tubulaire (1), de manière à empêcher la partie distale du deuxième support tubulaire (3) de sortir accidentellement de ladite cavité.

3. Prothèse selon la revendication 2, comprenant :
une bague de libération (11) positionnée autour dudit deuxième support tubulaire (3) de manière à coulisser au-dessus de celui-ci ;
un fil de libération (12) fixé à la bague de libération (11), qui passe à travers au moins un trou traversant (13) dans le capuchon (2) et s'étend longitudinalement le long de ladite paroi externe du troisième support tubulaire (5) ;
ledit fil de libération (12) et ladite bague de libération (12) étant conçus de sorte que, en tirant sur le fil de libération (12), la bague de libération (11) se déplace de manière longitudinale entrant dans la cavité définie par le capuchon (2) et soulevant le doigt de butée élastique (10) de ladite première crémaillère (9) pour permettre à la partie distale du deuxième support tubulaire (3) de sortir de ladite cavité.

4. Prothèse selon la revendication 1, dans laquelle ledit capuchon (2) ouvert comme un parapluie, ayant ladite butée mécanique, a une seconde crémaillère (17) définie sur une face interne du capuchon (2) et conçue pour venir en prise avec la première crémaillère (9) en faisant tourner axialement ledit deuxième support tubulaire (3) axialement sur lui-même par rapport audit capuchon (2).

5. Prothèse selon l'une des revendications précédentes, dans laquelle ledit capuchon (2) est vissé sur ledit premier support tubulaire (1).

6. Prothèse selon l'une des revendications précédentes, dans laquelle lesdites premières ailettes (4) et lesdites secondes ailettes (6) sont en Nitinol et sont conçues pour se déplier radialement dès qu'elles sont libres de se déplier.

7. Prothèse selon l'une des revendications précédentes, dans laquelle lesdites surfaces en regard respectives (7, 8) desdites ailettes (4, 6) ont des dents et/ou des harpons (14) permettant d'attraper des clapets d'une valve tricuspide ou mitrale.

8. Prothèse selon l'une des revendications précédentes, comprenant en outre un fil de guidage chirurgical (15) inséré dans le trou axial longitudinal du premier support tubulaire (1).

9. Prothèse selon l'une des revendications précédentes, dans laquelle ladite première pluralité d'ailettes (4) comprend trois premières ailettes espacées angulairement de 120°, et dans laquelle ladite seconde pluralité d'ailettes (6) comprend trois secondes ailettes espacées angulairement de 120°.

10. Dispositif de réparation d'une valve tricuspide ou mitrale, comprenant :
une prothèse selon l'une des revendications précédentes ;
un premier cathéter relié de manière amovible audit premier support tubulaire (1) ;
un deuxième cathéter relié de manière amovible audit deuxième support tubulaire (3) ;
un troisième cathéter relié de manière amovible audit troisième support tubulaire (5) ;
un quatrième cathéter (16), conçu pour coulisser longitudinalement sur la paroi externe dudit troisième support tubulaire (5) et sur les ailettes de ladite seconde pluralité d'ailettes (6) dans la configuration rétractée respective.

11. Dispositif selon la revendication précédente, dans lequel ledit quatrième cathéter (16) est conçu pour venir en butée contre un bord libre dudit capuchon (2) ouvert comme un parapluie de la prothèse.
